# FASCICULE DE BREVET EUROPEEN

(11) **EP 1 258 476 B1**
(45) Date de publication et mention de la délivrance du brevet: **28.06.2006**
(21) Numéro de dépôt: 02291192.9
(22) Date de dépôt: 14.05.2002
(51) Int. Cl.: C07D 207/16, C07D 277/04, C07D 295/18, C07D 277/06, A61K 31/40, A61K 31/426, A61P 3/10

(54) **Dérivés d'alpha-amino-acides, leur procédé de préparation ainsi que leur utilisation en tant qu'inhibiteurs de dipeptidyl-peptidase IV (DPP IV)**
Alpha-Aminosäure-Derivate, Verfahren zu deren Herstellung sowie deren Verwendung als Dipeptidylpeptidase-IV Inhibitoren (DPP IV)
Alpha-amino acid derivatives, method for their preparation and their use as dipeptidyl-peptidase IV inhibitors (DPP IV)

(30) Priorité: 15.05.2001 FR 0106375
(43) Date de publication de la demande: 20.11.2002
(73) Titulaire: Les Laboratoires Servier, 92200 Neuilly sur Seine (FR)
(72) Inventeur: de Nanteuil, Guillaume, 92150 Suresnes (FR); Portevin, Bernard, 78990 Elancourt (FR); Benoist, Alain, 95130 Franconville (FR); Levens, Nigel, 92420 Vaucresson (FR); Nosjean, Olivier, 92500 Rueil-Malmaison (FR); Husson-Robert, Bernadette, 92000 Nanterre (FR); Boulanger, Michelle, 78400 Chatou (FR)

(56) Documents cités:
- WO-A-95/15309
- WO-A-96/27593
- JIANG J D ET AL: "Inhibition of uman immunodeficiency virus type 1 infection in a T-cell line (CEM) by new dipeptidyl-peptidase IV (CD26) inhibitors" RESEARCH IN VIROLOGY, ELSEVIER, PARIS, FR, vol. 4, no. 148, 1 juillet 1997 (1997-07-01), pages 255-266, XP002074032 ISSN: 0923-2516
- YAMADA M ET AL: "A Potent Dipeptide Inhibitor of Dipeptidyl Peptidase IV" BIOORGANIC & MEDICINAL CHEMISTRY LETTERS, OXFORD, GB, vol. 8, no. 12, 16 juin 1998 (1998-06-16), pages 1537-1540, XP004137079 ISSN: 0960-894X
- LI J ET AL: "AMINOACYLPYRROLIDINE-2-NITRILES: POTENT AND STABLE INHIBITORS OF DEIPEPTIDYL-PEPTIDASE 4 (CD 26)" ARCHIVES OF BIOCHEMISTRY AND BIOPHYSICS, NEW YORK, US, US, vol. 323, no. 1, octobre 1995 (1995-10), pages 148-154, XP000965067 ISSN: 0003-9861
- AUGUSTYNS K ET AL: "THE UNIQUE PROPERTIES OF DIPEPTIDYL-PEPTIDASE IV (DPP IV/CD26) AND THE THERAPEUTIC POTENTIAL OF DPP IV INHIBITORS" CURRENT MEDICINAL CHEMISTRY, BENTHAM SCIENCE PUBLISHERS BV, BE, vol. 6, no. 4, 1999, pages 311-327, XP000870290 ISSN: 0929-8673
- ASHWORTH D M ET AL: "4-Cyanothiazolidides as very potent, stable inhibitors of dipeptidyl peptidase IV" BIOORGANIC & MEDICINAL CHEMISTRY LETTERS, OXFORD, GB, vol. 6, no. 22, 19 novembre 1996 (1996-11-19), pages 2745-2748, XP004135888 ISSN: 0960-894X

## Description

La présente invention concerne de nouveaux dérivés d'α-amino-acides, leur procédé de préparation, les compositions pharmaceutiques qui les contiennent ainsi que leur utilisation en tant qu'inhibiteurs de dipeptidyl-peptidase IV (DPP IV).

La dipeptidyl-peptidase IV est une sérine protéase membranaire, présente dans de nombreux tissus humains, et impliquée dans de nombreuses pathologies.

En particulier, il a été montré que la DPP IV était responsable de l'inactivation du GLP-1 (glucagon-like peptide-1). Or, le GLP-1 est un important stimulateur de la sécrétion d'insuline dans le pancréas et a donc un effet bénéfique direct sur le taux de glucose dans le sang.

L'inhibition de la DPP IV représente donc une approche extrêmement intéressante dans le traitement de l'intolérance au glucose et des maladies associées à l'hyperglycémie, telles que, par exemple, le diabète non insulino-dépendant (diabète de type II) ou l'obésité.

Des inhibiteurs de DPP IV ont déjà été décrits dans la littérature, notamment des dérivés d'amides dans la demande de brevet EP 0 490 379 et dans les journaux Current Med. Chem. 1999, 6 (4), 311-327, Arch. Biochem. Biophys. 1995, 323 (1), 148-154, Bioorg. Med. Chem. Lett. 1996, 6 (22), 2745-2748 et Adv. Exp. Med. Biol. 1997, 421, 157-160, et des dérivés de carbamates dans la demande de brevet DE 19826972.

D'autre part, des dérivés d'α-amino-acides, analogues de l'arginine, on été décrits dans la demande de brevet WO 96/27593 en tant qu'inhibiteurs de NO-synthase, utiles à ce titre pour le traitement de pathologies du système nerveux central et périphérique, de pathologies des dysfonctionnements du système gastrointestinal et urinaire, et de pathologies liées au système cardiovasculaire ou bronchopulmonaire.

Les composés de l'invention, eux, possèdent des propriétés inhibitrices de dipeptidyl-peptidase IV, qui les rendent donc particulièrement utiles pour le traitement de l'intolérance au glucose et des maladies associées à l'hyperglycémie.

Plus spécifiquement, la présente invention concerne les composés de formule (I) : 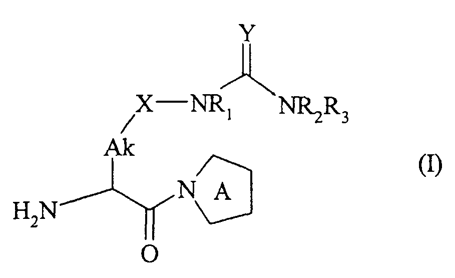 dans laquelle :
↘ 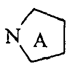 eprésente un hétérocycle azoté à 5 chaînons éventuellement substitué par un groupement cyano,
↘ Ak représente une chaîne alkylène (C₁-C₆) linéaire ou ramifiée,
↘ X représente une liaison simple ou un groupement phénylène,
↘ R₁ et R₂, identiques ou différents, représentent chacun un atome d'hydrogène ou un groupement alkyle (C₁-C₆) linéaire ou ramifié,
↘ Y représente NR₄ ou CH-NO₂,
   - R₄ représente un atome d'hydrogène ou un groupement alkyle (C₁-C₆) linéaire ou ramifié,
↘ R₃ représente :
   - soit un groupement choisi parmi alkyle (C₁-C₆) linéaire ou ramifié, nitro et cyano, dans le cas où Y représente CH-NO₂,
   - soit un groupement choisi parmi nitro et cyano, lorsque Y représente NR₄,
leurs tautomères lorsqu'ils existent, leurs isomères optiques et leurs sels d'addition à un acide pharmaceutiquement acceptable, à l'exclusion des composés pour lesquels, simultanément, 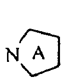 représente un hétérocycle azoté à 5 chaînons non substitué, Ak représente le groupement -(CH₂)₃-, X représente une liaison simple, Y représente NH et R₃ représente le groupement nitro.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthanesulfonique, camphorique, oxalique, etc.

Par hétérocycle azoté à 5 chaînons, on entend un groupement monocyclique saturé de 5 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre.

Les hétérocycles azotés à 5 chaînons préférés sont les groupements pyrrolidinyle et thiazolidinyle.

Les composés de formule (I) pour lesquels R₂ représente un atome d'hydrogène existent sous deux formes tautomères qui sont représentées par les formules (la) et (Ib) : 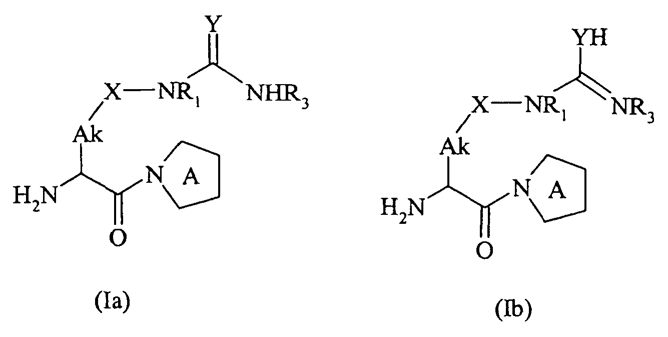 et qui font toutes deux partie intégrante de l'invention.

Les composés de formule (I) pour lesquels R₁ représente un atome d'hydrogène existent sous deux formes tautomères qui sont représentées par les formules (Ic) et (Id) : 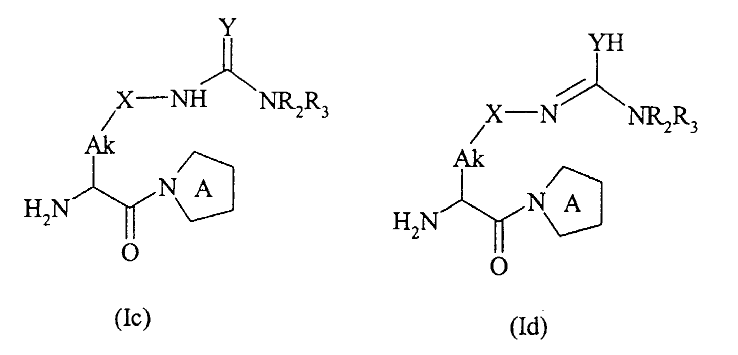 et qui font toutes deux partie intégrante de l'invention.

Les composés préférés de formule (I) sont ceux pour lesquels 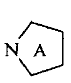 représente un groupement 1-pyrrolidinyle éventuellement substitué par un groupement cyano, ou 1,3-thiazolidin-3-yle éventuellement substitué par un groupement cyano.

Les composés préférés de formule (I) sont ceux pour lesquels la configuration en α de la fonction amide est (S).

Un aspect avantageux de l'invention concerne les composés de formule (I) pour lesquels

Ak représente le groupement (CH₂)₄.

Un autre aspect avantageux de l'invention concerne les composés de formule (I) pour lesquels 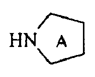 représente un groupement 1-pyrrolidinyle substitué par un groupement cyano, ou 1,3-thiazolidin-3-yle substitué par un groupement cyano, et Ak représente le groupement (CH₂)₃.

Un autre aspect avantageux de l'invention concerne les composés de formule (I) pour lesquels X représente une liaison simple.

Un autre aspect avantageux de l'invention concerne les composés de formule (I) pour lesquels Y représente un groupement NR₄, dans lequel R₄ représente un atome d'hydrogène ou un groupement alkyle (C₁-C₆) linéaire ou ramifié.

Un autre aspect avantageux de l'invention concerne les composés de formule (I) pour lesquels R₃ représente le groupement nitro.

Parmi les composés préférés de l'invention, on peut citer plus particulièrement :
- la N-{(4S)-4-amino-5-[(2S)-2-cyano-1-pyrrolidinyl]-5-oxopentyl}-N'-nitroguanidine, ses tautomères, ses isomères optiques ainsi que ses sels d'addition à un acide pharmaceutiquement acceptable,
- la N-{(5S)-5-amino-6-[(2S)-2-cyano-1-pyrrolidinyl]-6-oxohexyl}-N'-nitroguanidine, ses tautomères, ses isomères optiques ainsi que ses sels d'addition à un acide pharmaceutiquement acceptable,
- et la N-{(5S)-5-amino-6-(1,3-thiazolidin-3-yl)-6-oxohexyl}-N'-nitroguanidine, ses tautomères, ses isomères optiques ainsi que ses sels d'addition à un acide pharmaceutiquement acceptable,

L'invention s'étend également au procédé de préparation des composés de formule (I), caractérisé en ce que l'on met en réaction un composé de formule (II) : 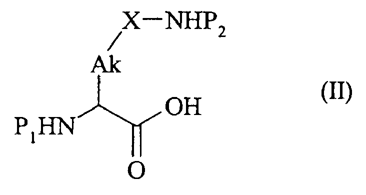 dans laquelle Ak et X sont tels que définis dans la formule (I), P₁ représente un groupement protecteur de la fonction amino, et P₂ représente un groupement protecteur de la fonction amino différent de P₁,
avec un composé de formule (III) : 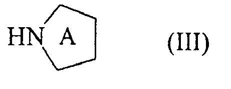 dans laquelle 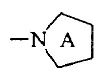 est tel que défini dans la formule (I),
dans des conditions classiques de couplage peptidique,
pour conduire après déprotection au composé de formule (IV), 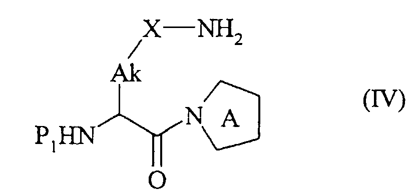 dans laquelle 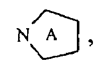 P₁, Ak et X sont tels que définis précédemment,
que l'on transforme ensuite, par des réactions classiques de la chimie organique, suivies d'une réaction de déprotection, en composé de formule (I),
que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

Les composés de formule (Ie), cas particulier des composés de formule (I): 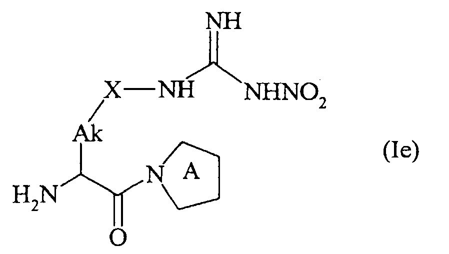 dans laquelle 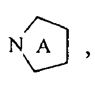 R₁, Ak et X sont tels que définis dans la formule (I),
peuvent également être obtenus à partir du composé de formule (V) : 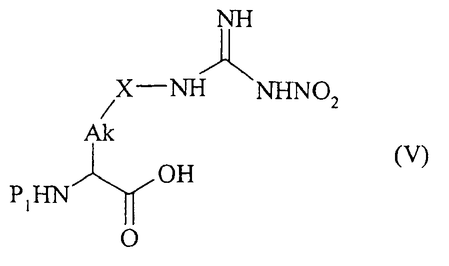 dans laquelle P₁, Ak et X sont tels que définis précédemment,
que l'on met en réaction avec le composé de formule (III), dans des conditions classiques de couplage peptidique,
pour conduire après déprotection éventuelle au composé de formule (Ie), que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

Les composés de la présente invention, outre le fait qu'ils soient nouveaux, présentent des propriétés pharmacologiques intéressantes. Ils ont des propriétés inhibitrices de la dipeptidyl-peptidase IV qui les rendent utiles dans le traitement de l'intolérance au glucose et des maladies associées à une hyperglycémie telles que le diabète de type II ou l'obésité.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse, intramusculaire ou sous-cutanée), nasale, les comprimés simples ou dragéifiés, les comprimés sublinguaux, les gélules, les tablettes, les suppositoires, les préparations injectables, les suspensions buvables, etc.

La posologie utile est adaptable selon la nature et la sévérité de l'affection, la voie d'administration ainsi que l'âge et le poids du patient et les traitements éventuellement associés. Cette posologie varie de 0,5 mg à 2 g par 24 heures en une ou plusieurs prises.

Les exemples suivants illustrent l'invention et ne la limitent en aucune façon.

Les produits de départ utilisés sont des produits connus ou préparés selon des modes préparatoires connus.

Les structures des composés décrits dans les exemples ont été déterminées selon les techniques spectrométriques usuelles (infrarouge, RMN, spectrométrie de masse).

Par composé de configuration (2α) ou (2β), on entend composé choisi parmi les composés de configurations absolues (2R) et (2S), étant entendu que lorsque le composé (2α) représente le composé de configuration absolue (2R), alors le composé (2β) représente le composé (2S).

### EXEMPLE 1 : N-{(4S)-4-Amino-5-[(2S)-2-cyano-1-pyrrolidinyl]-5-oxopentyl}-N'-nitroguanidine, chlorhydrate

### Stade A : N-{(4S)-4-[(Tert-butyloxycarbonyl)-amino]-5-[(2S)-2-cyano-1-pyrrolidinyl] -5-oxopentyl}-N'-nitroguanidine

A 10 mmoles de N²-(tert-butyloxycarbonyl)-N⁵-[(imino)-(nitroamino)-méthyl]-ornithine en solution dans la diméthylformamide sont ajoutées 10 mmoles de (2S)-2-cyano-pyrrolidine, 10 mmoles de 1-hydroxybenzotriazole et 10 mmoles de dicyclohexyl-carbodiimide. Après une nuit d'agitation à température ambiante, la dicyclohexylurée formée est filtrée, puis la diméthylformamide évaporée. Le résidu ainsi obtenu est purifié par chromatographie sur silice (éluant : dichlorométhane/éthanol 95/5) pour conduire au produit attendu.

### Stade B : N-{(4S)-4-Amino-5-[(2S)-2-cyano-1-pyrrolidinyl]-5-oxopentyl}-N'-nitroguanidine, chlorhydrate

A 10 mmoles du composé obtenu dans le stade précédent en solution dans le dioxane est ajoutée une solution 4N d'acide chlorhydrique dans le dioxane. Après 24 heures d'agitation à température ambiante, le solvant est évaporé, de l'eau est ajoutée et la solution lyophilisée pour conduire au produit attendu.

### Microanalyse élémentaire :

| | *C%* | *H%* | *N%* | *Cl%* |
|---|---|---|---|---|
| *calculé* | *39,58* | *6,04* | *29,37* | *10,62* |
| *trouvé* | *40, 35* | *6,00* | *29, 00* | *10,90* |

### EXEMPLE 2 : N-[(4S)-4-Amino-5-(1-pyrrolidinyl)-5-oxopentyl]-N'-cyanoguanidine, sesquichlorhydrate

### Stade A : 1-[(S)-N⁵-(Benzyloxycarbonyl)-N²-(tert-butyloxycarbonyl)-ornithyl]-pyrrolidine

Le produit attendu est obtenu selon le procédé décrit dans le stade A de l'exemple 1 à partir de (S)-N⁵-(benzyloxycarbonyl)-N²-(tert-butyloxycarbonyl)-ornithine et de pyrrolidine.

### Stade B : 1-[(S)-N²-(Tert-butyloxycarbonyl)-ornithyl]-pyrrolidine

10 mmoles du composé obtenu dans le stade précédent en solution dans l'éthanol sont hydrogénées en présence de palladium sur charbon à 10 %, à température et pression ambiantes, pendant 6 heures. Le milieu réactionnel est ensuite filtré et évaporé, puis, après addition d'eau, il est lyophilisé pour conduire au produit attendu.

### Stade C : N-{(4S)-4-[(Tert-butyloxycarbonyl)-amino]-5-(1-pyrrolidinyl)-5-oxopentyl}-N'-cyanoguanidine

Le produit attendu est obtenu selon le procédé décrit dans Synthesis 1975, 332, à partir du composé obtenu au stade précédent et de NaN(CN)₂.

### Stade D : N-[(4S)-4-Amino-5-(1-pyrrolidinyl)-5-oxopentyl]-N'-cyanoguanidine, sesquichlorhydrate

Le produit attendu est obtenu selon le procédé décrit dans le stade B de l'exemple 1 à partir du composé obtenu au stade précédent.

### Microanalyse élémentaire :

| | *C%* | *H%* | *N%* | *Cl%* |
|---|---|---|---|---|
| *calculé* | *43,03* | *7,06* | *27,37* | *17,32* |
| *trouvé* | *43, 26* | *7, 23* | *26, 82* | *16,85* |

### EXEMPLE 3 : N-[(4S)-4-Amino-5-(1,3-thiazolidin-3-yl)-5-oxopentyl]-N'-cyanoguanidine

### Stade A : 3-{(S)-N⁵-(Tert-butyloxycarbonyl)-N²-[(9H-fluorèn-9-yl-méthoxy)-carbonyl] -ornithyl}-1,3-thiazolidine

Le produit attendu est obtenu selon le procédé décrit dans le stade A de l'exemple 1 à partir de (S)-N⁵-(tert-butyloxycarbonyl)-N²-(9*H*-fluorèn-9-yl-méthoxy)-carbonyl]-ornithine et de 1,3-thiazolidine.

### Stade B : 3-{(S)-N²-[(9H-Fluorèn-9-yl-méthoxy)-carbonyl]-ornithyl}-1,3-thiazolidine, chlorhydrate

Le produit attendu est obtenu selon le procédé décrit dans le stade B de l'exemple 1 à partir du composé obtenu au stade précédent.

### Stade C : N-[(4S)-4-Amino-5-(1,3-thiazolidin-3-yl)-5-oxopentyl]-N'-cyanoguanidine

Le produit attendu est obtenu selon le procédé décrit dans Synthesis 1975, 332, à partir du composé obtenu au stade précédent et de NaN(CN)₂.

### Microanalyse élémentaire :

| | *C%* | *H%* | *N%* | *S%* |
|---|---|---|---|---|
| *calculé* | *44,43* | *6,71* | *31,08* | *11,86* |
| *trouvé* | *44,41* | *6,68* | *30,18* | *10,52* |

### EXEMPLE 4 : N-[(5S)-5-Amino-6-(1-pyrrolidinyl)-6-oxohexyl]-N'-cyanoguanidine, chlorhydrate

Le produit attendu est obtenu selon le procédé décrit dans l'exemple 2 en remplaçant la (S)-N⁵-(benzyloxycarbonyl)-N²-(tert-butyloxycarbonyl)-ornithine par la (S)-N⁶-(benzyloxycarbonyl)-N²-(tert-butyloxycarbonyl)-lysine.

### EXEMPLE 5 : N¹-[(4S)-4-Amino-5-(1-pyrrolidinyl)-5-oxopentyl]-N²-méthyl-2-nitro-1,1-éthylènediamine, dichlorhydrate

### Stade A : 1-[(S)-N⁵-(Benzyloxycarbonyl)-N²-(tert-butyloxycarbonyl)-ornithyl]-pyrrolidine

Le produit attendu est obtenu selon le procédé décrit dans le stade A de l'exemple 1 à partir de (S)-N⁵-(benzyloxycarbonyl)-N²-(tert-butyloxycarbonyl)-ornithine et de pyrrolidine.

### Stade B : 1-[(S)-N²-(Tert-butyloxycarbonyl)-ornithyl]-pyrrolidine

10 mmoles du composé obtenu dans le stade précédent en solution dans l'éthanol sont hydrogénées en présence de palladium sur charbon à 10 %, à température et pression ambiantes, pendant 6 heures. Le milieu réactionnel est ensuite filtré et évaporé, puis, après addition d'eau, il est lyophilisé pour conduire au produit attendu.

### Stade C : N¹-{(4S)-4-[(Tert-butyloxycarbonyl)-amino]-5-(1-pyrrolidinyl)-5-oxopentyl} -N²-méthyl-2-nitro-1,1-éthylènediamine

Le produit attendu est obtenu selon le procédé décrit dans Bioorg. Med. Chem. 1997, 7 (23), 3045-3048, à partir du composé obtenu au stade précédent et de N-méthyl-1-méthylthio-2-nitro-éthylènamine.

### Stade D : N1-[(4S)-4-Amino-5-(1-pyrrolidinyl)-5-oxopentyl]-N2-méthyl-2-nitro-1,1-éthylènediamine, dichlorhydrate

Le produit attendu est obtenu selon le procédé décrit dans le stade B de l'exemple 1 à partir du composé obtenu au stade précédent.

### Microanalyse élémentaire :

| | *C%* | *H%* | *N%* |
|---|---|---|---|
| *calculé* | *40,23* | *7,03* | *19,55* |
| *trouvé* | *40,17* | *6,98* | *18,92* |

### EXEMPLE 6 : N-[(4S)-4-Amino-5-(1-pyrrolidinyl)-5-oxopentyl]-N"-cyano-N'-méthylguanidine, chlorhydrate

### Stade A : 1-[(S)-N⁵-(Benzyloxycarbonyl)-N²-(tert-butyloxycarbonyl)-ornithyl]-pyrrolidine

Le produit attendu est obtenu selon le procédé décrit dans le stade A de l'exemple 1 à partir de (S)-N⁵-(benzyloxycarbonyl)-N²-(tert-butyloxycarbonyl)-ornithine et de pyrrolidine.

### Stade B : 1-[(S)-N²-(Tert-butyloxycarbonyl)-ornithyl]-pyrrolidine

10 mmoles du composé obtenu dans le stade précédent en solution dans l'éthanol sont hydrogénées en présence de palladium sur charbon à 10 %, à température et pression ambiantes, pendant 6 heures. Le milieu réactionnel est ensuite filtré et évaporé, puis, après addition d'eau, il est lyophilisé pour conduire au produit attendu.

### Stade C : N-{(4S)-4-[(Tert-butyloxycarbonyl)-amino]-5-(1-pyrrolidinyl)-5-oxopentyl}-N''-cyano-N'-méthylguanidine

Le produit attendu est obtenu selon le procédé décrit dans Chem. Pharm. Bull. 1997, 45 (1), 53-61, à partir du composé obtenu au stade précédent, de N-cyanoimido-S,S-diméthyldithiocarbonate et de méthylamine.

### Stade D : N-[(4S)-4-Amino-5-(1-pyrrolidinyl)-5-oxopentyl]-N''-cyano-N'-méthyl-guanidine, chlorhydrate

Le produit attendu est obtenu selon le procédé décrit dans le stade B de l'exemple 1 à partir du composé obtenu au stade précédent.
*Spectrométrie de masse : [M + H] + = 267 ; [M + Cl] + = 301 ; [M - H]- = 265.*

### EXEMPLE 7 : N-{(4S)-4-Amino-5-[(4R)-4-cyano-1,3-thiazolidin-3-yl]-5-oxopentyl}-N'-nitroguanidine, chlorhydrate

Le produit attendu est obtenu selon le procédé décrit dans l'exemple 1 en remplaçant la (2S)-2-cyano-pyrrolidine par la (4R)-4-cyano-1,3-thiazolidine.

### Microanalyse élémentaire :

| | *C%* | *H%* | *N%* | *S%* | *Cl%* |
|---|---|---|---|---|---|
| *calculé* | *34,14* | *5,16* | *27,87* | *9,11* | *10,08* |
| *trouvé* | *34,32* | *5,11* | *27,70* | *9,23* | *10,44* |

### EXEMPLE 8 : N-{(4S)-4-Amino-5-[(2α)-2-cyano-1,3-thiazolidin-3-yl]-5-oxopentyl}-N'-nitroguanidine, chlorhydrate

### Stade A : N-{(4S)-4-[(Tert-butyloxycarbonyl)-amino]-5-[2-carbamoyl-1,3-thiazolidin-3-yl]-5-oxopentyl}-N'-nitroguanidine, chlorhydrate

Le produit attendu est obtenu selon le procédé décrit dans le stade A de l'exemple 1, en remplaçant la (2S)-2-cyano-pyrrolidine par le (±)-1,3-thiazolidine-2-carboxamide.

### Stade B : N-{(4S)-4-[(Tert-butyloxycarbonyl)-amino]-5-[(2α)-2-carbamoyl-1,3-thiazolidin-3-yl]-5-oxopentyl}-N'-nitroguanidine, chlorhydrate

Le mélange de diastéréoisomères obtenu au stade A précédent est séparé par chromatographie sur silice (éluant : dichlorométhane/méthanol/NH₄OH 90/10/0,5).

Le produit attendu est le premier des diastéréoisomères ainsi séparés.

### Stade C : N-{(4S)-4-[(Tert-butyloxycarbonyl)-amino]-5-[(2α)-2-cyano-1,3-thiazolidin-3-yl]-5-oxopentyl}-N'-nitroguanidine

A 10 mmoles du composé obtenu au stade précédent en solution dans la pyridine sont ajoutées 20 mmoles d'imidazole, puis le mélange réactionnel est refroidi à -30°C, et 40 mmoles de POCl₃ sont additionnées au goutte à goutte. La température est ensuite amenée à -20°C en 1 heure. La pyridine est ensuite évaporée, et le résidu est dissout dans l'acétate d'éthyle. La solution est lavée, séchée, évaporée et le résidu obtenu est purifié par chromatographie sur silice (éluant : dichlorométhane/méthanol/NH₄OH 95/5/0,5) pour conduire au produit attendu.

### Stade D : N-{(4S)-4-Amino-5-[(2α)-2-cyano-1,3-thiazolidin-3-yl]-5-oxopentyl}-N'-nitroguanidine, chlorhydrate

Le produit attendu est obtenu selon le procédé décrit dans le stade B de l'exemple 1 à partir du composé obtenu au stade C précédent.

### Microanalyse élémentaire :

| | *C%* | *H%* | *N%* | *S%* | *Cl%* |
|---|---|---|---|---|---|
| *calculé* | *34,14* | *5,16* | *27,87* | *9,11* | *10, 08* |
| *trouvé* | *34,41* | *4,93* | *27, 67* | *9,35* | *9,71* |

### EXEMPLE 9 : N-{(4S)-4-Amino-5-[(2β)-2-cyano-1,3-thiazolidin-3-yl]-5-oxopentyl}-N'-nitroguanidine, chlorhydrate

Le produit attendu est obtenu selon le procédé décrit dans les stades C et D de l'exemple 8, à partir du deuxième des diastéréoisomères séparés au stade B de l'exemple 8.
*Spectrométrie de masse : LC*/*ESI*/*HR et MS*/*MS : [M + H]+ = 316*.

### EXEMPLE 10 : N-[(5S)-5-Amino-6-(1-pyrrolidinyl)-6-oxohexyl]-N'-nitroguanidine, chlorhydrate

Le produit attendu est obtenu selon le procédé décrit dans l'exemple 1 à partir de N²-(tert-butyloxycarbonyl)-N⁶-[(imino)-(nitroamino)-méthyl]-lysine et de pyrrolidine.

### EXEMPLE 11 : N-{(5S)-5-Amino-6-[(2S)-2-cyano-1-pyrrolidinyl]-6-oxohexyl}-N'-nitroguanidine, chlorhydrate

Le produit attendu est obtenu selon le procédé décrit dans l'exemple 1 à partir de N²-(tert-butyloxycarbonyl)-N⁶-[(imino)-(nitroamino)-méthyl]-lysine et de (2S)-2-cyano-pyrrolidine.

### Microanalyse élémentaire :

| | *C%* | *H%* | *N%* | *Cl%* |
|---|---|---|---|---|
| *calculé* | *41,44* | *6,38* | *28,19* | *10,19* |
| *trouvé* | *41,66* | *6,31* | *27,78* | *10,18* |

### EXEMPLE 12 : N-[(5S)-5-Amino-6-(1,3-thiazolidin-3-yl)-6-oxohexyl]-N'-nitroguanidine, chlorhydrate

Le produit attendu est obtenu selon le procédé décrit dans l'exemple 1 à partir de N²-(tert-butyloxycarbonyl)-N⁶-[(imino)-(nitroamino)-méthyl]-lysine et de 1,3-thiazolidine.
*Spectrométrie de masse : M + H] + = 305, M - H] - = 303*

### EXEMPLE 13 : N-{(5S)-5-Amino-6-[(4R)-4-cyano-1,3-thiazolidin-3-yl]-6-oxohexyl}-N'-nitroguanidine, chlorhydrate

Le produit attendu est obtenu selon le procédé décrit dans l'exemple 1 à partir de N²-(tert-butyloxycarbonyl)-N⁶-[(imino)-(nitroamino)-méthyl]-lysine et de (4R)-4-cyano-1,3-thiazolidine.

### Microanalyse élémentaire :

| | *C%* | *H%* | *N%* | *S%* | *Cl%* |
|---|---|---|---|---|---|
| *calculé* | *36,11* | *5,51* | *26,80* | *8,76* | *9, 69* |
| *trouvé* | *36,36* | *5,49* | *26,68* | *8,81* | *10,04* |

### EXEMPLE 14 : N-{(5S)-5-Amino-6-[(2α)-2-cyano-1,3-thiazolidin-3-yl]-6-oxohexyl}-N'-nitroguanidine, chlorhydrate

### Stade A : N-{(5S)-5-[(Tert-butyloxycarbonyl)-amino]-6-[2-carbamoyl-1,3-thiazolidin-3-yl]-6-oxohexyl}-N'-nitroguanidine, chlorhydrate

Le produit attendu est obtenu selon le procédé décrit dans le stade A de l'exemple 1 à partir de N²-(tert-butyloxycarbonyl)-N⁶-[(imino)-(nitroamino)-méthyl]-lysine et de (±)-1,3-thiazolidine-2-carboxamide.

### Stade B : N-{(5S)-5-[(Tert-butyloxycarbonyl)-amino]-6-[(2α)-2-carbamoyl-1,3-thiazolidin-3-yl]-6-oxohexyl}-N'-nitroguanidine, chlorhydrate

Le mélange de diatéréoisomères obtenu au stade A précédent est séparé par chromatographie sur silice (éluant : dichlorométhane / méthanol / NH₄OH 90/10/0,5). Le produit attendu est le premier des diastéréoisomères ainsi séparés.

### Stade C : N-{(5S)-5-Amino-6-[(2α)-2-cyano-1,3-thiazolidin-3-yl]-6-oxohexyl}-N'-nitroguanidine, chlorhydrate

Le produit attendu est obtenu selon le procédé décrit dans les stades C et D de l'exemple 8 à partir du composé obtenu au stade précédent.

### Microanalyse élémentaire :

| | *C%* | *H%* | *N%* | *S%* | *Cl%* |
|---|---|---|---|---|---|
| *calculé* | *36,11* | *5,51* | *26,80* | *8,76* | *9,69* |
| *trouvé* | *36,52* | *5,49* | *26,85* | *9, 26* | *9,51* |

### EXEMPLE 15 : N-{(5S)-5-Amino-6-[(2β)-2-cyano-1,3-thiazolidin-3-yl]-6-oxohexyl}-N'-nitroguanidine, chlorhydrate

Le produit attendu est obtenu selon le procédé décrit dans les stades C et D de l'exemple 8, à partir du deuxième des diastéréoisomères séparés au stade B de l'exemple 14.

### Microanalyse élémentaire :

| | *C%* | *H%* | *N%* | *S%* | *Cl%* |
|---|---|---|---|---|---|
| *calculé* | *36,11* | *5,51* | *26,80* | *8,76* | *9,69* |
| *trouvé* | *36,50* | *5,49* | *26, 00* | *9, 66* | *9, 66* |

### EXEMPLE 16 : N-{(4S)-4-Amino-5-[(2S)-2-cyano-1-pyrrolidinyl]-5-oxopentyl}-N'-cyanoguanidine, sesquichlorhydrate

Le produit attendu est obtenu selon le procédé décrit dans l'exemple 2 à partir de (S)-N⁵⁻(benzyloxycarbonyl)-N²-(tert-butyloxycarbonyl)-ornithine et de (2S)-2-cyano-pyrrolidine.

### Microanalyse élémentaire :

| | *C%* | *H%* | *N%* | *Cl%* |
|---|---|---|---|---|
| *calculé* | *43,03* | *7,06* | *27,37* | *17,32* |
| *trouvé* | *43,26* | *7, 23* | *26,82* | *16,85* |

### EXEMPLE 17: N-{(5S)-5-Amino-6-[(2S)-2-cyano-1-pyrrolidinyl]-6-oxohexyl}-N'-cyanoguanidine, chlorhydrate

Le produit attendu est obtenu selon le procédé décrit dans l'exemple 2 à partir de (S)-N⁶⁻(benzyloxycarbonyl)-N²-(tert-butyloxycarbonyl)-lysine et de (2S)-2-cyano-pyrrolidine.
*Spectrométrie de masse : M + H] + = 292, M - H J- = 290*

### EXEMPLE 18 : N-[(5S)-5-Amino-6-(1,3-thiazolidin-3-yl)-6-oxohexyl]-N'-cyanoguanidine, chlorhydrate

Le produit attendu est obtenu selon le procédé décrit dans l'exemple 2 à partir de (S)-N⁶⁻(benzyloxycarbonyl)-N²-(tert-butyloxycarbonyl)-lysine et de 1,3-thiazolidine.

### EXEMPLE 19 : N¹-[(4S)-4-Amino-5-(1,3-thiazolidin-3-yl)-5-oxopentyl]-N²-méthyl-2-nitro-1,1-éthylènediamine, dichlorhydrate

Le produit attendu est obtenu selon le procédé décrit dans l'exemple 5 à partir de (S)-N⁵⁻(benzyloxycarbonyl)-N²-(tert-butyloxycarbonyl)-ornithine et de 1,3-thiazolidine

### Microanalyse élémentaire :

| | *C%* | *H%* | *N%* | *S%* | *Cl%* |
|---|---|---|---|---|---|
| *calculé* | *35,11* | *6,16* | *18, 61* | *8,52* | *18,84* |
| *trouvé* | *35,54* | *5,99* | *18, 04* | *8, 61* | *19,51* |

### EXEMPLE 20 : N¹-[(5S)-5-Amino-6-(1-pyrrolidinyl)-6-oxohexyl]-N²-méthyl-2-nitro-1,1-éthylènediamine, dichlorhydrate

Le produit attendu est obtenu selon le procédé décrit dans l'exemple 5 à partir de (S)-N⁶⁻(benzyloxycarbonyl)-N²-(tert-butyloxycarbonyl)-lysine et de pyrrolidine.

### Microanalyse élémentaire :

| | *C%* | *H%* | *N%* | *Cl%* |
|---|---|---|---|---|
| *calculé* | *41,94* | *7,31* | *18,81* | *19,05* |
| *trouvé* | *41,87* | *7,26* | *18,34* | *19,75* |

### EXEMPLE 21 : N¹-{(5S)-5-Amino-6-[(2S)-2-cyano-pyrrolidinyl]-6-oxohexyl}-N²⁻méthyl-2-nitro-1,1-éthylènediamine, dichlorhydrate

Le produit attendu est obtenu selon le procédé décrit dans l'exemple 5 à partir de (S)-N⁶⁻(benzyloxycarbonyl)-N²-(tert-butyloxycarbonyl)-lysine et de (2S)-2-cyano-pyrrolidine.
*Spectrométrie de masse : ESI*/*FIA*/*HR et MS*/*MS : [M + H]* + *= 325 ; [M + Na]*+ *= 347.*

### EXEMPLE 22: N¹-[(5S)-5-Amino-6-(1,3-thiazolidin-3-yl)-6-oxohexyl]-N²-méthyl-2-nitro-1,1-éthylènediamine, dichlorhydrate

Le produit attendu est obtenu selon le procédé décrit dans l'exemple 5 à partir de (S)-N⁶⁻(benzyloxycarbonyl)-N²-(tert-butyloxycarbonyl)-lysine et de 1,3-thiazolidine.

### Microanalyse élémentaire :

| | *C%* | *H%* | *N%* | *S%* | *Cl%* |
|---|---|---|---|---|---|
| *calculé* | *36,93* | *6,46* | *17,94* | *8,22* | *18,17* |
| *trouvé* | *37,08* | *6,49* | *17,17* | *8,11* | *18,63* |

### EXEMPLE 23 : N-{(4S)-4-Amino-5-[(2S)-2-cyano-1-pyrrolidinyl]-5-oxopentyl}-N"-cyano-N'-méthylguanidine, chlorhydrate

Le produit attendu est obtenu selon le procédé décrit dans l'exemple 6 à partir de (S)-N⁵⁻(benzyloxycarbonyl)-N²-(tert-butyloxycarbonyl)-ornithine et de (2S)-2-cyano-pyrrolidine.

### EXEMPLE 24 : N-[(4S)-4-Amino-5-(1,3-thiazolidin-3-yl)-5-oxopentyl]-N"-cyano-N'-méthylguanidine, chlorhydrate

Le produit attendu est obtenu selon le procédé décrit dans l'exemple 6 à partir de (S)-N⁵⁻(benzyloxycarbonyl)-N²-(tert-butyloxycarbonyl)-ornithine et de 1,3-thiazolidine.

### EXEMPLE 25 : N-[(5S)-5-Amino-6-(1-pyrrolidinyl)-6-oxohexyl]-N"-cyano-N'-méthylguanidine, chlorhydrate

Le produit attendu est obtenu selon le procédé décrit dans l'exemple 6 à partir de (S)-N⁶⁻(benzyloxycarbonyl)-N²-(tert-butyloxycarbonyl)-lysine et de pyrrolidine.

### EXEMPLE 26 : N-{(5S)-5-Amino-6-[(2S)-2-cyano-1-pyrrolidinyl]-6-oxohexyl}-N"-cyano-N'-méthylguanidine, chlorhydrate

Le produit attendu est obtenu selon le procédé décrit dans l'exemple 6 à partir de (S)-N⁶⁻(benzyloxycarbonyl)-N²-(tert-butyloxycarbonyl)-lysine et de (2S)-2-cyano-pyrrolidine.

### EXEMPLE 27 : N-[(5S)-5-Amino-6-(1,3-thiazolidin-3-yl)-6-oxohexyl]-N"-cyano-N'-méthylguanidine, chlorhydrate

Le produit attendu est obtenu selon le procédé décrit dans l'exemple 6 à partir de (S)-N⁶⁻(benzyloxycarbonyl)-N²-(tert-butyloxycarbonyl)-lysine et de 1,3-thiazolidine.

### EXEMPLE 28 : N-{4-[(2S)-2-Amino-3-((2S)-2-cyano-1-pyrrolidinyl)-3-oxopropyl]-phényl}-N"-cyano-N'-méthylguanidine, chlorhydrate

Le produit attendu est obtenu selon le procédé décrit dans l'exemple 6 à partir de (S)-4-[(benzyloxycarbonyl)-amino]-N²-(tert-butyloxy-carbonyl)-phényl-alanine et de (2S)-2-cyano-pyrrolidine.

### EXEMPLE 29 : N-{4-[(2S)-2-Amino-3-oxo-3-(1-pyrrolidinyl)-propyl]-phényl}-N'-nitroguanidine, chlorhydrate

### Stade A : 4-[(2S)-2-[(Tert-butyloxycarbonyl)-amino]-3-oxo-3-(1-pyrrolidinyl)-propyl]-aniline

Le produit attendu est obtenu selon le procédé décrit dans le stade A de l'exemple 1 à partir d'acide (2S)-2-[(tert-butyloxycarbonyl)-amino]-3-(4-aminophényl)-propanoïque et de pyrrolidine.

### Stade B : N-{4-[(2S)-2-[(Tert-butyloxycarbonyl)-amino]-3-oxo-3-(1-pyrrolidinyl)-propyl]-phényl}-N'-nitroguanidine

Le produit attendu est obtenu par réaction du composé obtenu au stade précédent avec le N-méthyl-N'-2-dioxohydrazinecarboximidohydrazide-2-oxyde, selon le procédé décrit dans J. Am. Chem. Soc. 1949, 71, 1968-1970.

### Stade C : N-{4-[(2S)-2-Amino-3-oxo-3-(1-pyrrolidinyl)-propyl]-phényl}-N'-nitroguanidine, chlorhydrate

Le produit attendu est obtenu selon le procédé décrit dans le stade B de l'exemple 1 à partir du composé obtenu au stade B précédent.

### Microanalyse élémentaire :

| | *C%* | *H%* | *N%* | *Cl%* |
|---|---|---|---|---|
| *calculé* | *47,13* | *5,93* | *23,55* | *9,94* |
| *trouvé* | *48, 06* | *5,77* | *23, 60* | *10, 00* |

### EXEMPLE 30 : N-[(6S)-6-Amino-7-(1,3-thiazolidin-3-yl)-7-oxoheptyl]-N'-nitroguanidine, dichlorhydrate

Le produit attendu est obtenu selon le procédé décrit dans l'exemple 29 à partir d'acide (2S)-2-[(tert-butyloxycarbonyl)-amino]-7-amino-heptanoïque et de 1,3-thiazolidine.

### Microanalyse élémentaire :

| | *C%* | *H%* | *N%* | *S%* | *Cl* |
|---|---|---|---|---|---|
| *calculé* | *34,73* | *6, 28* | *22, 09* | *8,43* | *15,84* |
| *trouvé* | *34,78* | *6,19* | *21,24* | *8,58* | *15,56* |

### EXEMPLE 31 : N-[(6S)-6-Amino-7-[(2S)-2-cyano-1-pyrrolidinyl]-7-oxoheptyl]-N'-nitroguanidine, chlorhydrate

Le produit attendu est obtenu selon le procédé décrit dans l'exemple 29 à partir d'acide (2S)-2-[tert-butyloxycarbonyl)-amino]-7-amino-heptanoïque et de (2S)-2-cyano-pyrrolidine.

### Microanalyse élémentaire :

| | *C%* | *H%* | *N%* | *Cl%* |
|---|---|---|---|---|
| *calculé* | *43,15* | *6, 69* | *27,10* | *9,80* |
| *trouvé* | *43,75* | *6,59* | *26,93* | *9,94* |

### EXEMPLE 32 : N-{4-[(2S)-2-Amino-3-oxo-3-[(2S)-2-cyano-1-pyrrolidinyl]-propyl]-phényl}-N'-nitroguanidine, chlorhydrate

Le produit attendu est obtenu selon le procédé décrit dans l'exemple 29 à partir d'acide (2S)-2-[(tert-butyloxycarbonyl)-amino]-3-(4-aminophényl)-propanoïque et de (2S)-2-cyano-pyrrolidine.

### ETUDE PHARMACOLOGIQUE DES COMPOSES DE L'INVENTION

### EXEMPLE 33 : Inhibition de la dipeptidyl-peptidase IV in vitro

L'effet des composés sur l'activité enzymatique *in vitro* de la DPPIV est évalué comme suit. L'enzyme de rein de porc est dosée à l'aide d'un substrat chromogène, le glycyl-prolyl*-p*-nitroanilide 1,4 mM, qui est hydrolysé pour libérer de la *p*-nitroaniline absorbant à 405 nm. L'activité de l'enzyme est déterminée par absorbance, en présence de concentrations variables du composé à évaluer (10⁻⁴ à 10⁻⁹ M le plus souvent). Les données obtenues permettent de déterminer la dose efficace pour l'inhibition à 50 % de l'activité contrôle (IC₅₀). Les composés de l'invention possèdent une IC₅₀ comprise entre 1nM et 10 µM.

### EXEMPLE 34 : Composition pharmaceutique

### Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 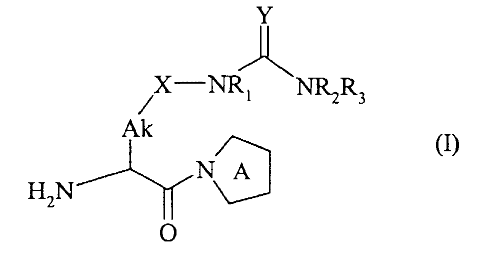 dans laquelle :
↘ 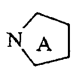 représente un hétérocycle azoté à 5 chaînons éventuellement substitué par un groupement cyano,
↘ Ak représente une chaîne alkylène (C₁-C₆) linéaire ou ramifiée,
↘ X représente une liaison simple ou un groupement phénylène,
↘ R₁ et R₂, identiques ou différents, représentent chacun un atome d'hydrogène ou un groupement alkyle (C₁-C₆) linéaire ou ramifié,
↘ Y représente NR₄ ou CH-NO₂,
• R₄ représente un atome d'hydrogène ou un groupement alkyle (C₁-C₆) linéaire ou ramifié,
↘ R₃ représente :
- soit un groupement choisi parmi alkyle (C₁-C₆) linéaire ou ramifié, nitro et cyano, dans le cas où Y représente CH-NO₂,
- soit un groupement choisi parmi nitro et cyano, lorsque Y représente NR₄,
ses tautomères lorsqu'ils existent, ses isomères optiques et ses sels d'addition à un acide pharmaceutiquement acceptable,
à l'exclusion des composés pour lesquels, simultanément, 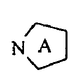 représente un hétérocycle azoté à 5 chaînons non substitué, Ak représente le groupement -(CH₂)₃-, X représente une liaison simple, Y représente NH et R₃ représente le groupement nitro,
étant entendu que par hétérocycle azoté à 5 chaînons, on entend un groupement monocyclique saturé de 5 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre.

2. Composé de formule (I) selon la revendication 1 tel que 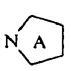 représente un groupement 1-pyrrolidinyle éventuellement substitué par un groupement cyano ou 1,3-thiazolidin-3-yle éventuellement substitué par un groupement cyano.

3. Composé de formule (I) selon l'une quelconque des revendications 1 ou 2 tel que la configuration en α de la fonction amide est (S).

4. Composé de formule (I) selon l'une quelconque des revendications 1 à 3 tel que Ak représente le groupement (CH₂)₄.

5. Composé de formule (I) selon l'une quelconque des revendications 1 à 3 tel que 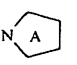 représente un groupement 1-pyrrolidinyle substitué par un groupement cyano, ou 1,3-thiazolidin-3-yle substitué par un groupement cyano, et Ak représente le groupement (CH₂)₃.

6. Composé de formule (I) selon l'une quelconque des revendications 1 à 5 tel que X représente une liaison simple.

7. Composé de formule (I) selon l'une quelconque des revendications 1 à 6 tel que Y représente un groupement NR₄, dans lequel R₄ représente un atome d'hydrogène ou un groupement alkyle (C₁-C₆) linéaire ou ramifié.

8. Composé de formule (I) selon l'une quelconque des revendications 1 à 7 tel que R₃ représente le groupement nitro.

9. Composé de formule (I) selon la revendication 1 qui est la N-{(4S)-4-amino-5-[(2S)-2-cyano-1-pyrrolidinyl]-5-oxopentyl}-N'-nitroguanidine, ainsi que ses isomères optiques, ses tautomères et ses sels d'addition à un acide pharmaceutiquement acceptable.

10. Composé de formule (I) selon la revendication 1 qui est la N-{(5S)-5-amino-6-[(2S)-2-cyano-1-pyrrolidinyl]-6-oxohexyl}-N'-nitroguanidine, ainsi que ses isomères optiques, ses tautomères et ses sels d'addition à un acide pharmaceutiquement acceptable.

11. Composé de formule (I) selon la revendication 1 qui est la N-{(5S)-5-amino-6-(1,3-thiazolidin-3-yl)-6-oxohexyl-N'-nitroguanidine, ainsi que ses isomères optiques, ses tautomères et ses sels d'addition à un acide pharmaceutiquement acceptable.

12. Procédé de synthèse des composés de formule (I) selon la revendication 1, **caractérisé en ce que** l'on met en réaction un composé de formule (II) : 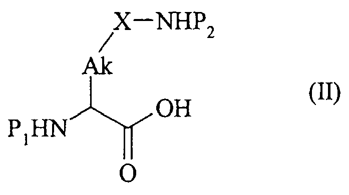 dans laquelle Ak et X sont tels que définis dans la formule (I), P₁ représente un groupement protecteur de la fonction amino, et P₂ représente un groupement protecteur de la fonction amino différent de P₁,
avec un composé de formule (III) : 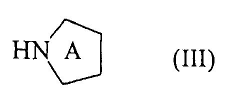 dans laquelle 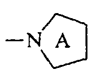 est tel que défini dans la formule (I),
dans des conditions classiques de couplage peptidique,
pour conduire après déprotection au composé de formule (IV), 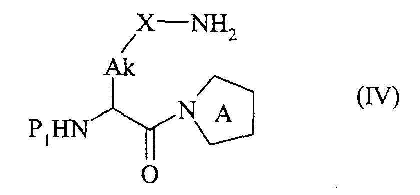 dans laquelle 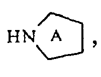 P₁, Ak et X sont tels que définis précédemment,
que l'on transforme ensuite, par des réactions classiques de la chimie organique, suivies, d'une réaction de déprotection, en composé de formule (I),
que l'on purifie, le cas échéant, selon une technique classique de purification, dont on sépare, si on le souhaite, les isomères optiques selon une technique classique de séparation, et que l'on transforme, si on le souhaite, en leurs sels d'addition à un acide pharmaceutiquement acceptable.

13. Composition pharmaceutique contenant comme principe actif un composé selon l'une quelconque des revendications 1 à 11, en combinaison avec un ou plusieurs véhicules inertes, non toxiques et pharmaceutiquement acceptables.

14. Composition pharmaceutique selon la revendication 13 utile en tant que médicament comme inhibiteur de dipeptidyl-peptidase IV, utile dans le traitement de l'intolérance au glucose, et des maladies associées à une hyperglycémie telles que le diabète de type II ou l'obésité.

15. Composition pharmaceutique selon la revendication 14 utile en tant que médicament antidiabétique.

## Claims

1. Compound of formula (I) : 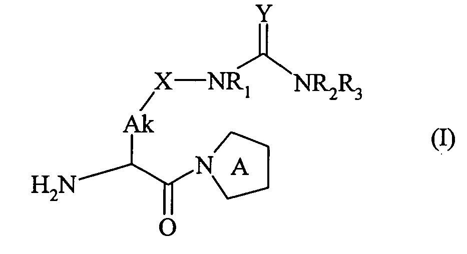 wherein:
↘ 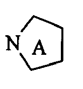 represents a 5-membered nitrogen-containing heterocycle optionally substituted by a cyano group,
↘ Ak represents a linear or branched (C₁-C₆)alkylene chain,
↘ X represents a single bond or a phenylene group,
↘ R₁ and R₂, which may be identical or different, each represent a hydrogen atom or a linear or branched (C₁-C₆)alkyl group,
↘ Y represents NR₄ or CH-NO₂,
• R₄ represents a hydrogen atom or a linear or branched (C₁-C₆)alkyl group,
↘ R₃ represents :
- a group selected from linear or branched (C₁-C₆)alkyl, nitro and cyano, when Y represents CH-NO₂, or
- a group selected from nitro and cyano, when Y represents NR₄,
its tautomers when they exist, its optical isomers, and addition salts thereof with a pharmaceutically acceptable acid,
with the exclusion of compounds wherein, simultaneously, 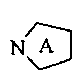 represents an unsubstituted 5-membered nitrogen-containing heterocycle, Ak represents the group -(CH₂)₃-, X represents a single bond, Y represents NH and R₃ represents the nitro group,
it being understood that the term "5-membered nitrogen-containing heterocycle" is understood to mean a 5-membered saturated monocyclic group containing one, two or three hetero atoms, one of which hetero atoms is the nitrogen atom, and any additional hetero atoms present are selected from the atoms oxygen, nitrogen and sulphur.

2. Compound of formula (I) according to claim 1 wherein 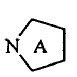 represents a 1-pyrrolidinyl group optionally substituted by a cyano group, or a 1,3-thiazolidin-3-yl group optionally substituted by a cyano group.

3. Compound of formula (I) according to either claim 1 or claim 2 wherein the configuration α to the amide function is (S).

4. Compound of formula (I) according to any one of claims 1 to 3 wherein Ak represents the group (CH₂)₄.

5. Compound of formula (I) according to any one of claims 1 to 3 wherein 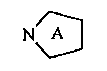 represents a 1-pyrrolidinyl group substituted by a cyano group, or a 1,3-thiazolidin-3-yl group substituted by a cyano group, and Ak represents the group (CH₂)₃.

6. Compound of formula (I) according to any one of claims 1 to 5 wherein X represents a single bond.

7. Compound of formula (I) according to any one of claims 1 to 6 wherein Y represents a group NR₄, wherein R₄ represents a hydrogen atom or a linear or branched (C₁-C₆)alkyl group.

8. Compound of formula (I) according to any one of claims 1 to 7 wherein R₃ represents the nitro group.

9. Compound of formula (I) according to claim 1 which is N-{(4S)-4-amino-5-[(2S)-2-cyano-1-pyrrolidinyl]-5-oxopentyl}-N'-nitroguanidine, and its optical isomers, its tautomers, and addition salts thereof with a pharmaceutically acceptable acid.

10. Compound of formula (I) according to claim 1 which is N-{(5S)-5-amino-6-[(2S)-2-cyano-1-pyrrolidinyl]-6-oxohexyl}-N'-nitroguanidine, and its optical isomers, its tautomers, and addition salts thereof with a pharmaceutically acceptable acid.

11. Compound of formula (I) according to claim 1 which is N-{(5S)-5-amino-6-(1,3-thiazolidin-3-yl)-6-oxohexyl}-N'-nitroguanidine, and its optical isomers, its tautomers, and addition salts thereof with a pharmaceutically acceptable acid.

12. Process for the synthesis of compounds of formula (I) according to claim 1, **characterised in that** a compound of formula (II) : 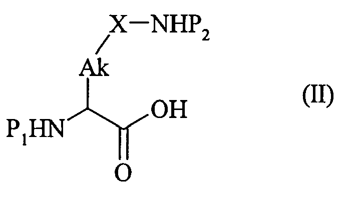 wherein Ak and X are as defined for formula (I), P₁ represents an amino-function-protecting group, and P₂ represents an amino-function-protecting group other than P₁,
is reacted with a compound of formula (III) : 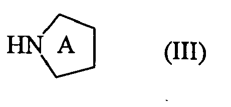 wherein 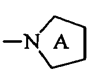 is as defined for formula (I),
under conventional peptide coupling conditions,
to yield, after deprotection, a compound of formula (IV), 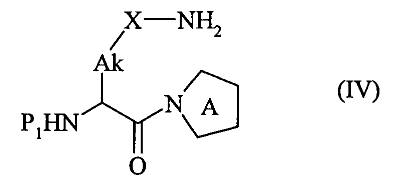 wherein 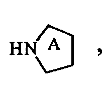 P₁, Ak and X are as defined hereinbefore,
which is then converted, by conventional organic chemistry reactions, followed by a deprotection reaction, to a compound of formula (I),
which is purified, if necessary, according to a conventional purification technique, separated, if desired, into its optical isomers according to a conventional separation technique, and converted, if desired, into addition salts thereof with a pharmaceutically acceptable acid.

13. Pharmaceutical composition comprising as active ingredient a compound according to any one of claims 1 to 11, in combination with one or more inert, non-toxic pharmaceutically acceptable carriers.

14. Pharmaceutical composition according to claim 13 for use as a medicament as a dipeptidyl peptidase IV inhibitor, for use in the treatment of glucose intolerance and of disorders associated with hyperglycaemia, such as type II diabetes or obesity.

15. Pharmaceutical composition according to claim 14 for use as an antidiabetic medicament.

## Patentansprüche

1. Verbindung der Formel (I): 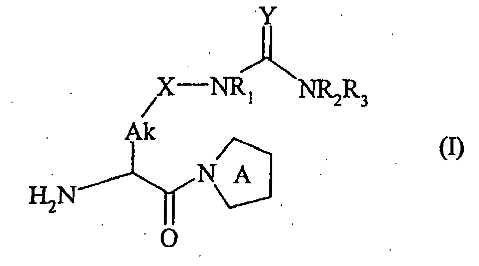 in der:
↘ 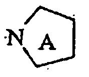 einen stickstoffhaltigen Heterocyclus mit 5 Kettengliedern bedeutet, der gegebenenfalls durch eine Cyanogruppe substituiert ist,
↘ Ak eine geradkettige oder verzweigte (C₁-C₆)-Alkylenkette bedeutet,
↘ X eine Einfachbindung oder eine Phenylengruppe bedeutet,
↘ R₁ und R₂, die gleichartig oder verschieden sind, jeweils ein Wasserstoffatom oder eine geradkettige oder verzweigte (C₁-C₆)-Alkylgruppe bedeuten,
↘ Y NR₄ oder CH-NO₂ bedeutet, worin
• R₄ ein Wasserstoffatom oder eine geradkettige oder verzweigte (C₁-C₆)-Alkylgruppe darstellt,
↘ R₃:
- entweder eine Gruppe ausgewählt aus geradkettigem oder verzweigtem (C₁-C₆)-Alkyl, Nitro und Cyano bedeutet, für den Fall, da Y CH-NO₂ darstellt,
- oder eine Gruppe ausgewählt aus Nitro und Cyano bedeutet, wenn Y NR₄ darstellt,
ihre Tautomeren, falls sie existieren, ihre optischen Isomeren und ihre Additionssalze mit einer pharmazeutisch annehmbaren Säure, mit Ausnahme der Verbindungen, bei denen gleichzeitig 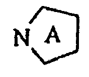 einen nichtsubstituierten stickstoffhaltigen Heterocyclus mit 5 Kettengliedern darstellt, Ak die Gruppe -(CH₂)₃- darstellt, X eine Einfachbindung darstellt, Y NH darstellt und R₃ die Nitrogruppe darstellt,
wobei es sich versteht, daß man unter einem stickstoffhaltigen Heterocyclus mit 5 Kettengliedern eine monocyclische gesättigte Gruppe mit 5 Kettengliedern versteht, die eines, zwei oder drei Heteroatome enthält, wobei eines dieser Heteroatome das Stickstoffatom ist und das oder die gegebenenfalls vorhandenen weiteren Heteroatome aus Sauerstoff-, Stickstoff- und Schwefelatomen ausgewählt sind.

2. Verbindung der Formel (I) nach Anspruch 1, worin 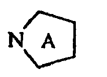 eine gegebenenfalls durch eine Cyanogruppe substituierte 1-Pyrrolidinylgruppe oder eine gegebenenfalls durch eine Cyanogruppe substituierte 1,3-Thiazolidin-3-yl-gruppe bedeutet.

3. Verbindung der Formel (I) nach einem der Ansprüche 1 oder 2, worin die Konfiguration in der α-Stellung zu der Amidfunktion die (S)-Konfiguration ist.

4. Verbindung der Formel (I) nach einem der Ansprüche 1 bis 3, worin Ak die Gruppe (CH₂)₄ bedeutet.

5. Verbindung der Formel (I) nach einem der Ansprüche 1 bis 3, worin 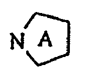 eine durch eine Cyanogruppe substituierte 1-Pyrrolidinylgruppe oder durch eine Cyanogruppe substituierte 1,3-Thiazolidin-3-yl-gruppe bedeutet und Ak die Gruppe (CH₂)₃ darstellt.

6. Verbindung der Formel (I) nach einem der Ansprüche 1 bis 5, worin X eine Einfachbindung bedeutet.

7. Verbindung der Formel (I) nach einem der Ansprüche 1 bis 6, worin Y eine Gruppe NR₄ bedeutet, worin R₄ ein Wasserstoffatom oder eine geradkettige oder verzweigte (C₁-C₆)-Alkylgruppe darstellt.

8. Verbindung der Formel (I) nach einem der Ansprüche 1 bis 7, worin R₃ die Nitrogruppe bedeutet.

9. Verbindung der Formel (I) nach Anspruch 1, nämlich N-{(4S)-4-Amino-5-[(2S)-2-cyano-1-pyrrolidinyl]-5-oxopentyl}-N'-nitroguanidin sowie dessen optische Isomere, dessen Tautomere und dessen Additionssalze mit einer pharmazeutisch annehmbaren Säure.

10. Verbindung der Formel (I) nach Anspruch 1, nämlich N-{(5S)-5-Amino-6-[(2S)-2-cycano-1-pyrrolidinyl]-6-oxohexyl}-N'-nitroguanidin und dessen optische Isomere, dessen Tautomere und dessen Additionssalze mit einer pharmazeutisch annehmbaren Säure.

11. Verbindung der Formel (I) nach Anspruch 1, nämlich N-{(5S)-5-Amino-6-(1,3-thiazolidin-3-yl)-6-oxohexyl}-N'-nitroguanidin sowie dessen optische Isomere, dessen Tautomere und dessen Additionssalze mit einer pharmazeutisch annehmbaren Säure.

12. Verfahren zur Synthese der Verbindungen der Formel (I) nach Anspruch 1, **dadurch gekennzeichnet, daß** man eine Verbindung der Formel (II): 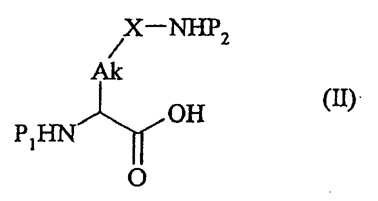 in der Ak und X die bezüglich der Formel (I) angegebenen Bedeutungen besitzen, P₁ eine Schutzgruppe für die Aminofunktion darstellt und P₂ eine von P₁ verschiedene Schutzgruppe für die Aminofunktion darstellt,
mit einer Verbindung der Formel (III): 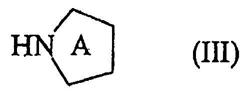 in der 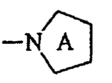 die bezüglich der Formel (I) angegebenen Bedeutungen besitzt,
bei den klassischen Bedingungen der Peptidkupplung umsetzt,
so daß man nach der Abspaltung der Schutzgruppe die Verbindung der Formel (IV) erhält: 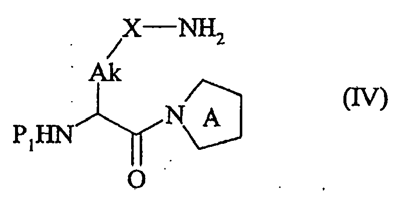 in der 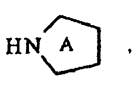 P₁, Ak und X die oben angegebenen Bedeutungen besitzen,
welche man anschließend mit Hilfe klassischer Reaktionen der organischen Chemie, gefolgt von einer Reaktion zur Abspaltung der Schutzgruppen, in die Verbindung der Formel (I) umwandelt,
welche man gegebenenfalls mit Hilfe einer klassischen Reinigungsmethode reinigt, die man gewünschtenfalls mit Hilfe einer klassischen Trennmethode in ihre optischen Isomeren auftrennt und welche man gewünschtenfalls in ihre Additionssalze mit einer pharmazeutisch annehmbaren Säure überführt.

13. Pharmazeutische Zubereitung enthaltend als Wirkstoff eine Verbindung nach einem der Ansprüche 1 bis 11 in Kombination mit einem oder mehreren inerten, nichttoxischen und pharmazeutisch annehmbaren Trägermaterialien.

14. Pharmazeutische Zubereitung nach Anspruch 13, nützlich als Arzneimittel mit inhibierender Wirkung auf die Dipeptidyl-peptidase IV, nützlich bei der Behandlung der Glucoseunverträglichkeit, von Erkrankungen, die mit einer Hyperglykämie verknüpft sind, wie Diabetes Typ II oder Fettsucht.

15. Pharmazeutische Zubereitung nach Anspruch 14, nützlich als antidiabetisches Arzneimittel.
